# EUROPEAN PATENT SPECIFICATION

(11) **EP 3 731 899 B1**
(45) Date of publication and mention of the grant of the patent: **23.10.2024**
(21) Application number: 18830808.4
(22) Date of filing: 20.12.2018
(51) Int. Cl.: A61M 5/20

(54) **MOUNTING ADAPTER FOR INJECTION DEVICE AND ADD-ON DEVICE**
MONTAGEADAPTER FÜR INJEKTIONSVORRICHTUNG UND ZUSATZVORRICHTUNG
ADAPTATEUR DE MONTAGE POUR DISPOSITIF D'INJECTION ET DISPOSITIF COMPLÉMENTAIRE

(30) Priority: 28.12.2017 EP 17306952
(43) Date of publication of application: 04.11.2020
(73) Proprietor: SANOFI, 75008 Paris (FR)
(72) Inventor: HELMER, Michael, 65926 Frankfurt am Main (DE)
(74) Representative: Weilnau, Carsten
(86) International application number: PCT/EP2018/086101
(87) International publication number: WO 2019/129620

(56) References cited:
- WO-A1-2010/037828
- WO-A1-2010/037828
- WO-A1-2017/021228
- WO-A1-2017/021228
- WO-A2-2010/128493
- WO-A2-2010/128493
- DE-U1- 29 904 864
- DE-U1- 29 904 864

## Description

### Field

The present disclosure relates to a mounting adapter for mounting an add-on device or supplemental device to an injection device. In another aspect the disclosure relates to an add-on device for an injection device.

### Background

Drug delivery devices for setting and dispensing a single or multiple doses of a liquid medicament are as such well-known in the art. Generally, such devices have substantially a similar purpose as that of an ordinary syringe.

Drug delivery devices, such as pen-type injectors have to meet a number of user-specific requirements. For instance, with patient's suffering chronic diseases, such like diabetes, the patient may be physically infirm and may also have impaired vision. Suitable drug delivery devices especially intended for home medication therefore need to be robust in construction and should be easy to use. Furthermore, manipulation and general handling of the device and its components should be intelligible and easy understandable. Such injection devices should provide setting and subsequent dispensing of a dose of a medicament of variable size. Moreover, a dose setting as well as a dose dispensing procedure must be easy to operate and has to be unambiguous.

Typically, such devices comprise a housing or a particular cartridge holder, adapted to receive a cartridge at least partially filled with the medicament to be dispensed. The device further comprises a drive mechanism, usually having a displaceable piston rod to operably engage with a bung or piston of the cartridge. By means of the drive mechanism and its piston rod, the bung or piston of the cartridge is displaceable in a distal or dispensing direction and may therefore expel a predefined amount of the medicament via a piercing assembly, e.g. in form of an injection needle, which is to be releasably coupled with a distal end section of the housing of the drug delivery device.

The medicament to be dispensed by the drug delivery device is provided and contained in a multi-dose cartridge. Such cartridges typically comprise a vitreous barrel sealed in distal direction by means of a pierceable seal and being further sealed in proximal direction by the piston. With reusable drug delivery devices an empty cartridge is replaceable by a new one. In contrast to that, drug delivery devices of disposable type are to be entirely discarded when the medicament in the cartridge has been dispensed or used-up.

With some drug delivery devices, such as pen-type injection devices a user has to set a dose of variable size by rotating a dose dial and a dose dial sleeve in a clockwise or dose-incrementing direction relative to a body or housing of the injection device. For injecting and expelling of a dose of a liquid medicament the user will have to depress a trigger or dose button in a distal direction and hence towards the body or housing of the injection device. Typically, the user uses his thumb for exerting a distally directed pressure onto the dose button, which is located at a proximal end of the dose dial and the dose dial sleeve, while holding the housing of the injection device with the remaining fingers of the same hand.

For mechanically implemented injection devices it is desirable to enable a precise, reliable and quasi automated supervising and/or collection of injection-related data during use of the injection device. Mechanically operated injection devices may be equipped with an electronically implemented add-on device or data collection device configured to monitor user-induced operation of the injection device. A data collection device for attachment to an injection device should be rather compact with regards to its geometric size. The mechanical attachment of add-on devices or data collecting device to injection devices is rather challenging. In order to provide a precise and reliable monitoring of e.g. a mechanically implemented injection device the add-on device must be precisely positioned, e.g. on an outside surface of the injection device.

Document WO 2010/037828 A1 discloses a drug delivery device and a monitoring device adapted to detect an action taking place in the drug delivery device. The monitoring device comprises a mounting portion with an opening for receiving a monitoring portion.

### Objects

It is therefore an object to provide improve mechanical connection of an add-on device to an injection device. The mechanical connection should be universally applicable to a large variety of injection devices without the necessity to modify the geometry or structure of the injection device. The mechanical connection should provide a reliable, stable and long-lasting fastening of the add-on device to the injection device to enable a precise measuring and monitoring of injection-related parameters. Moreover, the mechanical connection should be easy to manufacture in a mass production process. It should be rather space saving and should support an energy-efficient monitoring or tracking of the operation of the injection device.

### Summary

In one aspect there is provided a mounting adapter configured for mounting an add-on device to an injection device. The mounting adapter, which is defined in claim 1, comprises a carrier and a first mount connected to the carrier. The first mount is configured to connect to a housing of the injection device. The mounting adapter further comprises a second mount that is connected to the carrier as well. By means of the first mount the mounting adapter itself is connectable or attachable to the housing of the injection device. By means of the second mount the add-on device is attachable and connectable to the mounting adapter. Since the first mount and the second mount are both connected to the carrier the add-on device is attachable and connectable to the injection device through and by way of the mounting adapter.

The mounting adapter further comprises an electrical conductor arranged in or on the carrier. The electrical conductor is configured to exchange at least one type of electric signals with the add-on device. Implementation of the electric conductor into a mounting adapter is beneficial in many aspects. A rather spacious electrical conductor, e.g. in form of an antenna or in form of a capacitive electrode can be arranged outside and remote from the add-on device and may be electrically connectable to the add-on device or to electrical components thereof through the mounting adapter. In this way, rather spacious and comparatively large-scaled electrical conductors can be arranged outside the add-on device. This enables to reduce the geometric size of the add-on device thus increasing a degree of user acceptance and increasing an ease of use of such add-on devices.

The mounting adapter is further beneficial in order to enable mounting of one and the same add-on device to different injection devices. For this, different types of mounting adapters each of which comprising a specifically implemented first mount complementary-shaped to differently-shaped or differently sized housings of different injection devices can be provided. In this way, one and the same type of add-on device can be used with a large variety of injection devices. For attaching the add-on device to an injection device add-on device-specific modifications to the injection device become superfluous; and vice versa.

The mounting adapter further comprises at least a first electrical contact connected to the electrical conductor. The at least first electrical contact is arranged on at least one of the second mount, an upper side of the carrier, an upper side of an electric layer and an upper side of a cover layer. Here, the optional electric layer is arranged on the upper side of the carrier. The optional cover layer extends over a major portion of the upper side of the carrier.

According to a further example the carrier of the mounting adapter comprises a sheet of an elastic foil. The sheet of elastic foil is at least one of pliable, elastically deformable or stretchable. In this way, the carrier is configured to adapt or to conform to the geometric shape of the housing of the injection device. The first mount can be integrally formed with the carrier. Hence, a portion of the carrier may constitute the first mount. In further examples the first mount may extend across or over a major portion of the carrier. The first mount may also extend over the entire carrier, typically over an underside of the carrier. In further examples the carrier may comprise a rather stiff and rigid fastening element, such as a clamp or clip configured to enclose at least a portion of the housing of the injection device.

According to another example the first mount comprises an adhesive layer arranged on or extending across an underside of the carrier. The underside of the carrier is opposite to the upper side of the carrier. The adhesive layer may comprise or may be constituted by an adhesive agent applied to the underside of the carrier. The adhesive layer may also extend across the entirety of the underside of the carrier. This enables an adhesive attachment of the carrier to the housing of the injection device, typically to an outside surface of the housing of the injection device. An adhesive layer and hence an adhesive attachment of the carrier to the housing of the injection device is universally applicable to differently sized or differently configured housings of a variety of different injection devices. An adhesive attachment does not require any modification to the geometry or construction of the injection device.

According to another example the electrical conductor is directly arranged in or on the layer. The electrical conductor can be a printed electrically conductive structure. It may be printed or directly adhered on an upper side of the carrier. In further examples the electrical conductor is implemented inside the carrier. Here, an electrical conductor may be embedded in the carrier. The carrier may comprise a laminated foil enclosing the electrical conductor. With other examples the carrier may comprise an injection molded plastic material with the electrical conductor embedded therein.

According to another example the mounting adapter further comprises the electric layer. The electric layer comprises a sheet on an electrically insulating foil arranged on an upper side of the carrier. Here, the electrical conductor is arranged on the electrically insulating foil. The electrical conductor is arranged on at least one of an underside of the insulating foil and an upper side of the insulating foil. Typically, the underside of the insulating foil faces towards an upper side of the carrier. An upper side of the insulating foil faces away from the carrier. The electrical conductor can be provided on at least one of the upper side and underside of the insulating foil. When the electrical conductor is arranged on the underside of the electrically insulating foil the electrical conductor may get in direct electrical contact with an upper side of the carrier.

With such examples the carrier itself is made of an electrically insulating material, such as plastic. It may comprise a plastic foil. Also, the electrically insulating foil may comprise a pliable, bendable or stretchable plastic foil on which the electrical conductor is printed. With other examples the electrical conductor is exclusively provided on an upper side of the electrically insulating foil. With such examples the first mount does not have to be of electrically insulating type. With this example, the carrier may comprise or may be formed of a sheet of metal.

With further examples it is conceivable, that the electrical conductor or that several electrical conductors are arranged on both opposite sides of the electrically insulating foil. One or a first electrical conductor may be arranged on an upper side of the electrically insulating foil. A second electrical conductor can be arranged on the underside of the electrically insulating foil. The first and the second electrical conductors may be electrically isolated from each other. They may be electrically disconnected from each other. The separate first and second electrical conductors may serve different purposes. A first conductor may comprise or form an electrode for measuring at least one physical parameter of the injection device. The second electrical conductor may comprise or constitute an antenna configured for a wireless transmission of data and/or wireless capturing or harvesting of electromagnetic energy. In this way, the mounting adapter may serve different electric purposes.

In further examples a first electrical conductor on an upper side of the carrier and a second electrical conductor on a lower side or underside of the electrically insulating foil are electrically interconnected. In this way a ratio of an effective length of the electrical conductor versus the geometric dimension or size of the electrically insulating foil can be increased, e.g. by a factor 2.

According to another example the mounting adapter further comprises the cover layer. The cover layer extends over at least a major portion of an upper side of the carrier and covering the electrical conductor. The cover layer may be combined with different configurations of the mounting adapter. In examples where the mounting adapter is void of the electric layer and hence void of a sheet of an electrically insulating foil, the cover layer may be directly attached or arranged on the upper side of the carrier, thereby also covering the electrical conductor provided on the upper side of the carrier.

Here, the electrical conductor is located between the cover layer and the upper side of the carrier. The cover layer is typically of electrically insulating type. The cover layer may comprise or may be constituted by a pliable, elastic or stretchable plastic foil. In any example the cover layer may comprise an outer surface facing away from the carrier located underneath. The upper or outer surface of the cover layer may serve as an information surface. The outer surface of the cover layer may be provided with visual indications or visual information, e.g. regarding the type of medicament contained in the mounting adapter or regarding instructions of use regarding the medicament, the injection device and/or the add-on device.

Insofar, the cover layer provides a twofold function. It may provide information to the user and may therefore comprise an information label. The cover layer may comprise a printed label. According to another aspect the cover layer serves to protect the electrical conductor underneath and may seal and protect the upper side of the carrier against environmental influences or hazards. In particular, the information label may contain or comprise regulatory information about the drug device combination product which is formed by the injection device with a medicament container located therein.

In a further example the mounting adapter further comprises at least a second electrical contact connected to the electrical conductor or connected to a second electrical conductor. The second electrical conductor may arranged in or on the carrier and the second electrical conductor may be also configured to exchange at least one type of electric signals with the add-on device. The at least second electrical contact is arranged on at least one of the second mount, an upper side of the carrier, an upper side of the electric layer and an upper side of the cover layer.

When there are provided several electrical conductors, such as a first electrical conductor and a second electrical conductor there is also provided a respective number of electrical contacts. For instance, with a first and with a second electrical conductor there are provided first and second electrical contacts. The first electrical contact is connected to the first electrical conductor and the second electrical contact is connected to the second electrical conductor.

Both, the first and the second electrical contacts are arranged on the same or on different sides of at least one of the second mount, the upper side of the carrier, the upper side of the electric layer and the upper side of the cover layer. In this way, the at least first or several electrical contacts are accessible from outside the mounting adapter. They are in particular accessible to and for the add-on device when the add-on device is appropriately mounted on the mounting adapter. By means of the at least first electrical contact or by means of several electrical contacts, an electrical connection between the electrical conductor and the add-on device can be established. Typically, the add-on device comprises at least a first correspondingly- or complementary-shaped electrical device contact that gets in direct mechanical and electrical contact with the at least first electrical contact of the mounting adapter when the add-on device is correctly mounted on the mounting adapter.

In examples, wherein the mounting adapter is void of a cover layer and wherein the electric layer it is intended that the at least first electrical contact is arranged on at least one of the second mount and an upper side of the carrier. When the mounting adapter comprises the adhesive layer the at least first electrical contact is arranged on at least one of the second mount, the upper side of the carrier and the upper side of the electric layer. When the mounting adapter comprises a cover layer but is void of the electric layer the at least first electrical contact is arranged on at least one of the second mount, an upper side of the carrier and an upper side of the cover layer.

The at least first electrical contact may be of rather flat shape and may be entirely located in a plane in which the respective electrical conductor extends. In other examples the at least first electrical contact may protrude from at least one of a surface of the carrier, the electric layer and the cover layer. The first electrical contact may comprise a mechanical contact structure, such as a plug or socket. In other examples the at least first electrical contact may comprise an enlarged surface portion having lateral dimensions that exceed at least one dimension or cross-section of a portion of the electrical conductor. Spatially enlarged electrical contacts are beneficial to establish and electrical connection to the add-on device when the mechanical connection between the mounting adapter and the add-on device should be subject to manufacturing or mounting tolerances. At least a cross-section or a dimension of the electrical contacts is larger than manufacturing or mounting tolerances of at least one of the mounting adapter and the add-on device.

When the mounting adapter comprises a multilayer structure, e.g. when there is provided at least one of an electric layer and a cover layer on the carrier, the cover layer and/or the electric layer may comprise a through opening, through which the electrical conductor or through which the at least first electrical contact extends. It is even conceivable, that the second mount comprises a recessed portion or a through opening, through which at least one of the electrical conductor and the at least first electrical contact extends. In this way the electrical conductor buried inside or embedded in a multilayer structure of the mounting adapter can become accessible from outside the mounting adapter.

According to another example the second mount comprises a rigid mounting base. The second mount may comprise an injection molded plastic material connected to the carrier. The carrier may comprise a pliable or elastic plastic foil welded to the second mount. In some examples the carrier and the second mount may be integrally formed. They may be both made of a single injection molded plastic component.

In a further example the rigid mounting base comprises a foot and a neck. Here, the foot is connected to the carrier and the neck extends through at least one of the electric layer and the cover layer. In embodiments wherein the mounting adapter is void of the electric layer and the cover layer the neck simply protrudes from an outside surface of the carrier. The foot may be integrally formed with the carrier. When the carrier and the rigid mounting base are provided as separate components, the foot may be rigidly attached to the carrier, e.g. by an adhesive or welded connection.

Typically, the protruding neck comprises a cross-section that is smaller than a cross-section of the foot. The foot may comprise an extension along the surface of the carrier that exceeds a respective dimension of the neck with regard to the same direction. Typically, the neck is narrowed compared to the foot. The neck may comprise a longitudinal protrusion or a protruding stud or pin configured to mechanically engage with a correspondingly-shaped mount of the add-on device.

With some examples the rigid mounting base comprises a foot, a narrowed neck and a widened head. A cross-section of the head is typically larger than a cross-section of the neck. The head is arranged on an end of the neck facing away from the foot. The neck is located between the widened head and the foot.

A widened foot is of particular benefit to provide a durable, mechanically resistant and long lasting mechanical connection to the carrier, e.g. by way of an adhesive or welded connection. A head featuring a cross-section extending a cross-section of the neck is beneficial to enable a form fitting fastening of the add-on device to the second mount of the mounting adapter. The rigid mounting base, e.g. formed of an injection molded plastic material, such as a thermoplastic material, e.g. polypropylene, polyethylene or polyamide. The injection moldable plastic material might be of biodegradable type. It may may comprise or consist of a plastic material from a bioilogical source. The injection moldable plastic material provides a well-defined, e.g. a slack-free detachable fastening of the add-on device to the mounting adapter.

In some examples it is of particular benefit, when the rigid mounting base is provided on the upper side of the carrier and below at least one of the underside of the electric layer and the underside of the cover layer. For instance, at least one of the cover layer and the electric layer may comprise a recess configured to receive the neck of the rigid mounting base there through. Here, an inner diameter or cross-section of the recess in at least one of the electric layer and the cover layer is substantially identical to an outer diameter or outer cross-section of the neck.

The recess, in particular its inner dimensions may be smaller than the outer dimensions of the foot of the rigid mounting base. In this way the electric layer or cover layer may cover at least a portion of the foot when attached to the carrier. A fastening of the rigid mounting base to the carrier can be further improved by at least one of the electric layer and cover layer when at least one of the electric layer and cover layer is sufficiently fastened to the carrier.

At least one of the cover layer and electric layer or both may comprise a longitudinal slit forming a recess to receive the neck of the rigid mounting base. For this, the slit may comprise a longitudinal slit extending into a side edge of at least one of the electric layer and cover layer. The longitudinal slit may comprise a constant slit width along its longitudinal extension. Such a slit shape is beneficial for a neck having a constant cross-section along the longitudinal extension of the slit or recess of at least one of the electric layer and the cover layer.

According to another example the electrical conductor comprises or constitutes at least one of a capacitive electrode and an antenna. The electrical conductor may comprise both, a capacitive electrode and an antenna. The electrical conductor may comprise a first electrically conductive structure and a second electrically conductive structure. The first and the second electrically conductive structures may be provided on the same side of the electric layer or on opposite sides of the electric layer. The first and the second electrically conductive structures may be both connected to at least one or two electrical contacts. The at least one electrical conductor may comprise opposite ends, each end being connected to a respective electrical contact. In this way, the electrical conductor may form a conductor loop that extends along a surface of at least one of the carrier and the electric layer.

When implemented as an antenna the electrical conductor may be configured to transfer electrical signals between the add-on device and an external electronic device. The antenna may be implemented as a radio-frequency antenna. It may enable a wireless data communication between the add-on device and an external electronic device. The external electronic device may comprise a portable electronic device, such as a smartphone, a smartwatch or a tablet computer. The antenna may be configured as a near field communication (NFC) antenna that is based on the Radio-Frequency Identification Standard (RFID). In other examples, the antenna may be configured for wireless communication according to other wireless communication standards. Only as an example the antenna may be implemented as a WLAN antenna or Wi-Fi antenna in accordance to the IEEE-802.11 standard.

When the add-on device is connected to the mounting adapter the antenna of the mounting adapter may exchange electrical signals with a communication unit of the add-on device. The antenna may be implemented as an antenna for transceiving electrical data signals. In other embodiments or examples the antenna may be configured to harvest or to capture electromagnetic energy from a surrounding electromagnetic field. It is even conceivable, that the electrical conductor comprises a first and a second antenna. A first antenna may be configured as an electric energy harvesting antenna whereas the second antenna may be exclusively configured to transceive or to exchange data with an external electronic device.

In further examples the electrical conductor comprises a capacitive electrode. In some examples the electrical conductor may comprise at least two or even more capacitive electrodes arranged at a predefined distance, e.g. on the electric layer. The capacitive electrode or the capacitive electrodes may be arranged in such a way, that different portions of the capacitive electrodes or that electrically insulated capacitive electrodes are located at diametrically opposite portions of a medicament reservoir contained inside the injection device. Such capacitive electrodes enable a quantitative measuring of the content of a medicament container.

By means of at least one or several capacitive electrodes an amount of a liquid medicament contained in a medicament container can be determined. The regular arrangement, the liquid medicament is located between two or more capacitive electrodes. In this way, the electrical conductor may be connected to a sensor arrangement of the add-on device via the respective electrical contacts when the add-on device is appropriately connected to the mounting adapter and when the mounting adapter is appropriately connected to the housing of the injection device. The amount of medicament located between capacitive electrodes is directly correlated to a measurable electrical capacity between the electrodes. In this way, a rather effective and space saving solution is provided to enable a fill level measuring of a medicament container located inside the housing of the injection device.

Simply by attaching the mounting adapter to the injection device the respective measuring electrodes are in a required position in order to conduct the respective measurement. The measuring electrodes or capacitive electrodes are thus in close proximity to the medicament container. The medicament container may for instance comprise a vitreous cartridge filled with the liquid medicament. Such a cartridge may comprise a rather elongated shape and may extend all along a cartridge holder section of the housing of the injection device. By attaching the mounting adapter all along the cartridge holder or at least at a dedicated circumferential section thereof the respective measuring electrodes may extend along the longitudinal extension of the medicament cartridge. Such a rather spacious arrangement could not be implemented into the add-on device. By arranging such measuring or capacitive electrodes in the mounting adapter no modifications are necessary to the injection device to enable such a capacity-based fill level determination of fill level measuring of the cartridge. The implementation of a measuring electrode or a capacitive electrode in the mounting adapter therefore enables to reduce the size of the add-on device.

According to another aspect the disclosure also relates to an add-on device configured for monitoring the operation of an injection device. Said add-on device is defined in claim 9. The monitoring device comprises a housing with a third mount. The third mount is configured to mechanically engage with the second mount of a mounting adapter as described above.

The second mount and the third mount are configured to form or to constitute a mechanical connection. The second mount and the third mount may be correspondingly- or complementary-shaped. They may be mechanically encoded. Hence, the second mount may comprise a second mechanical code and the third mount may comprise a third mechanical code that match mutually. Another add-on device with a non-matching code cannot be attached or connected to the mounting adapter. At least one of the second mount and the third mount may comprise a plug and the other one of the second mount and the third mount may comprise a correspondingly- or complementary-shaped socket. In other examples at least one of the first mount and the second mount comprises a protrusion and the other one of the second mount and the third mount comprises a correspondingly-shaped recess.

According to another example the add-on device comprises a memory and a processor connected to the at least first electrical device contact. The memory and the processor may be arranged on a printed circuit board (PCB). The memory may be integrated into the processor. The memory may comprise both a program memory and a main memory. The processor may for instance be a microprocessor, a Digital Signal Processor (DSP), Application Specific Integrated Circuit (ASIC), Field Programmable Gate Array (FPGA) or the like. The processor is configured to execute program code (e.g. software or firmware) stored in the program memory, and uses a main memory, for instance to store intermediate results. The main memory may also be used to store a logbook on performed ejections/injections. The program memory may for instance be a Read-Only Memory (ROM), and the main memory may for instance be a Random Access Memory (RAM).

By means of the at least first electrical device contact the processor and/or the memory is electrically connectable to the electrical conductor or to various electrical conductors of the mounting adapter. In this way, an electric connection between the processor and at least one of a measuring electrode and an antenna can be established by mounting the add-on device onto the mounting adapter.

According to another example the add-on device comprises a sensor arrangement. The sensor arrangement comprises at least one of an acceleration sensor, a position sensor, a capacity sensor, an optical sensor and a magnetic sensor. The sensor arrangement may be entirely enclosed in a body or in a housing of the add-on device. In some examples the sensor arrangement or at least a particular sensor thereof may be located outside the add-on device and may be interconnected with the add-on device via the mutually corresponding electric contacts. For instance, a capacity sensor, e.g. comprising one or numerous capacitive electrodes may be formed or constituted by at least one electrical conductor arranged in or on the carrier of the mounting adapter.

In another example the add-on device further comprises a communication unit connected to the at least first electrical device contact. The communication unit is configured to transceive at least one of electromagnetic signals and electromagnetic energy via the at least first electrical conductor of the mounting adapter when the at least first electrical contact is connected to the at least first electrical device contact. The mounting adapter and the add-on device may comprise numerous, hence first, second and third electrical contacts and respective electrical device contacts that mutually correspond to each other.

The communication unit is electrically connectable through the at least first electrical device contact with the electrical conductor of the mounting adapter. Here, the electrical conductor of the mounting adapter comprises an antenna or an antenna loop. The antenna or antenna loop typically comprises a first electrical contact and a second electrical contact configured to establish an electrical connection with respective first and second electrical device contacts. The communication unit is typically configured to transceive, i.e. to receive and to transmit electrical signals via the antenna. In this way, data captured and measured by the add-on device can be transmitted to at least one or several external electronic devices by way of a wireless transmission.

In another example the add-on device is void of an own battery or electric power supply. Here, the antenna may be configured to harvest electromagnetic energy from an external electromagnetic field, e.g. from an RF-electromagnetic field. Then, the antenna and hence the electrical conductor may be connected to an electric power unit of the add-on device that is configured to derive a driving current from the electromagnetic radiation, which drive current is operable to power up and to drive the processor of the add-on device. With this example the add-on device can be implemented as a passive add-on device that is void of an own power supply. Alternatively, the add-on device may be provided with a rechargeable battery of limited capacity supporting and allowing an emergency operation of the add-on device in cases where an external electromagnetic field should not be available. If an external electromagnetic is available the battery could be recharged.

According to another aspect the disclosure further relates to an injection system. The injection system comprises a mounting adapter as described above and an add-on device as described above. The injection system further comprises an injection device featuring and comprising a housing. Here, the first mount of the mounting adapter is connected to the housing of the injection device and the third mount of the add-on device is connected to the second mount of the mounting adapter. In this way, the add-on device is connectable and mountable to the injection device exclusively via the mounting adapter. With the injection system there may be provided a number of differently-shaped injection devices.

With each injection device there may be provided a correspondingly-shaped mounting adapter. Each of these mounting adapters is configured to conform to an outside surface of the respective injection device. Each of a set of differently configured or differently-shaped mounting adapters comprises a second mount that is identical with all mounting adapters of the set of mounting adapters. In this way, the add-on device is universally attachable to any of the injection devices which is equipped with a correspondingly shaped mounting adapter. In effect, one and the same add-on device can be used with a series of different mounting adapters provided that each mounting adapter comprises a respective mount specifically-shaped to connect to the third mount of the add-on device.

In another example the injection device comprises a medicament container that is filled with an injectable medicament. The medicament container may comprise a longitudinally-shaped cartridge, e.g. a tubular-shaped cartridge that is filled with an injectable medicament. The cartridge may be pre-filled with the medicament and the cartridge may be pre-assembled inside the injection device when the injection device becomes commercially available to the customers or patients.

In the present context the term 'distal' or distal end' relates to an end of the injection device that faces towards an injection site of a person or of an animal. The term 'proximal' or 'proximal end' relates to an opposite end of the injection device, which is furthest away from an injection site of a person or of an animal.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-GIn-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the scope of the claims.

Further, it is to be noted, that any reference numerals used in the appended claims are not to be construed as limiting the scope of the invention.

### Brief description of the drawings

In the following, numerous examples of the container and of an injection device will be described in greater detail by making reference to the drawings, in which:
- Fig. 1: is a schematic view of an injection device configured as a pen-type injector,
- Fig. 2: shows the injection device equipped with a mounting adapter,
- Fig. 3: shows the injection system with an add-on device connected to the injection device via the mounting adapter,
- Fig. 4: is indicative of the injection system with the add-on device attached to the injection device but with a removed cover layer thus exhibiting the electric layer or the electrical conductor of the mounting adapter,
- Fig. 5: schematically illustrates a housing of the add-on device comprising a third mount,
- Fig. 6: shows a bottom side of the add-on device according to Fig. 5,
- Fig. 7: schematically illustrates a multilayer structure of the mounting adapter,
- Fig. 8: shows a rigid mounting base of the mounting adapter in an enlarged view,
- Fig. 9: shows a detail of the rigid mounting base,
- Fig. 10: shows the mounting base arranged on the carrier,
- Fig. 11: is indicative of another multilayer structure of the mounting adapter,
- Fig. 12: shows another geometric profile of the mounting base,
- Fig. 13: is illustrative of intermediate product during the manufacturing of the mounting adapter and
- Fig. 14: shows a block diagram of the injection system with the various components of the mounting adapter and the add-on device.

### Detailed description

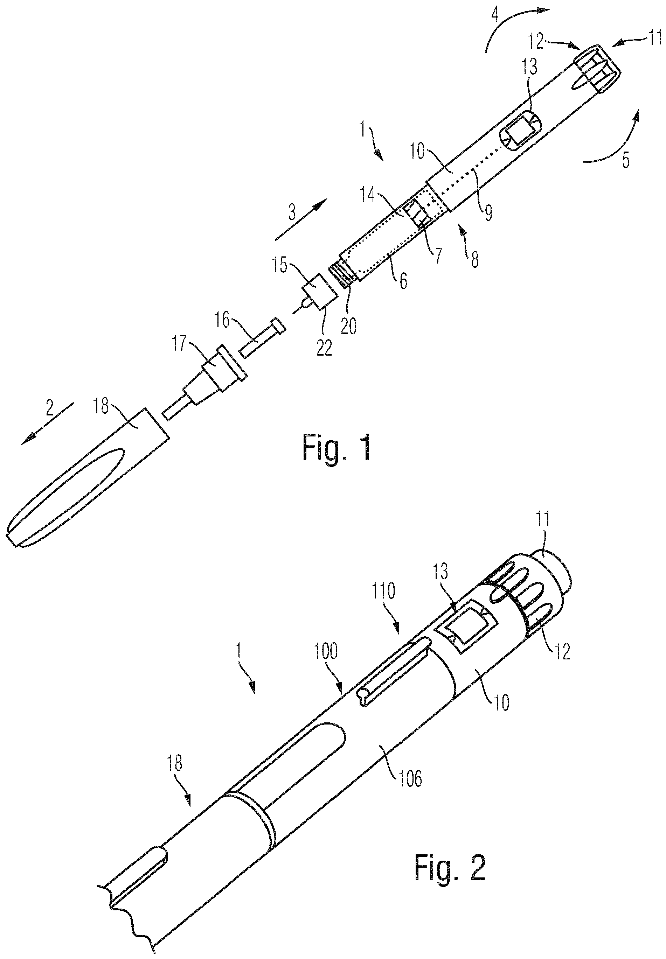

The injection device 1 as shown in Figs. 1 and 2 is a pre-filled disposable injection device that comprises a housing 10 to which a needle assembly 15 can be affixed. The injection needle 15 is protected by an inner needle cap 16 and either an outer needle cap 17 or a protective cap 18 that is configured to enclose and to protect a distal section of the housing 10 of the injection device 1. The housing 10 may comprise and form a main housing part configured to accommodate a drive mechanism 8. The injection device 1 may further comprise a distal housing component denoted as cartridge holder 14. The cartridge holder 14 may be permanently or releasably connected to the main housing 10. The cartridge holder 14 is typically configured to accommodate a cartridge 6 that is filled with a liquid medicament.

As indicated in Fig. 1 the injection device 1 comprises a housing 10 and a container 6 filled with a liquid medicament. The container 6 provides a medicament reservoir. It may comprise a substantially tubular-shaped barrel or bottle filled with the liquid medicament. The container 6 may comprise a cartridge. Towards a proximal direction 3 the medicament reservoir 6 may be closed by a displaceable bung 7 or stopper. The bung 7 may be in mechanical contact with a piston rod 9 configured for a stepwise distally directed displacement in order to urge the bung 7 further in distal direction 2 and hence towards a distal outlet of the medicament reservoir 6. At or near the outlet the medicament reservoir 6 may comprise a pierceable membrane. The needle assembly 15 comprises a threaded needle hub 22 configured for a screwed connection with the threaded socket 20 provided on a distal end of the cartridge holder 14. In this way, the injection needle gains access to the interior of the medicament container 6.

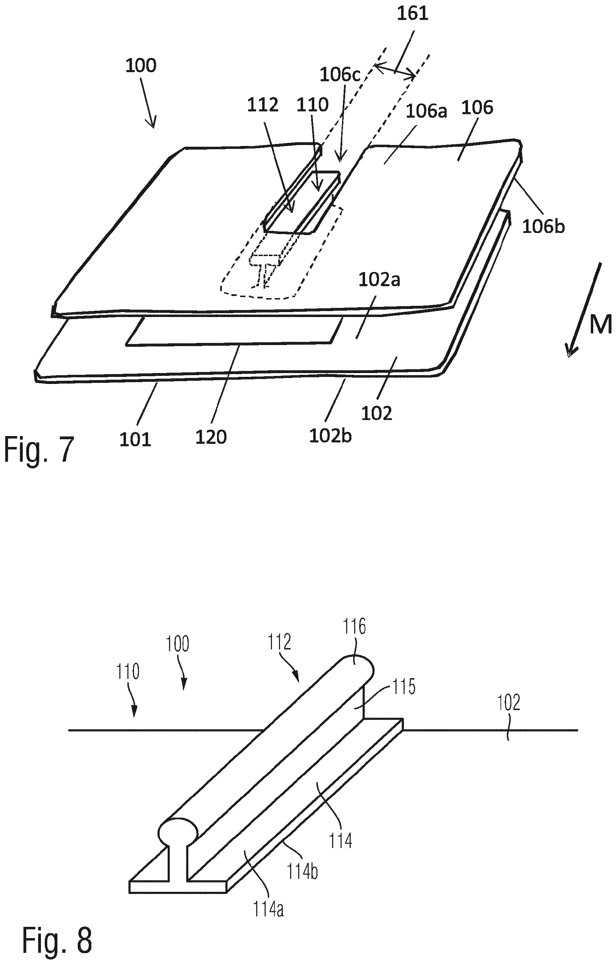

In Fig. 2 the injection device 1 is provided with a mounting adapter 100. The mounting adapter 100 is shown in more detail in Fig. 7. The mounting adapter 100 comprises a carrier 102, a first mount 101, a second mount 110 and an electrical conductor 120. As shown in Fig. 7 the carrier 102 comprises a substantially planar sheet of a pliable or flexible material. The carrier 102 may comprise a pliable plastic foil. The carrier 102 comprises an upper side 102a and an underside 102b. The electrical conductor 120 may comprise a conductor loop and may be printed as an electrically conductive structure on the upper side 102a of the carrier 102. In an alternative example the electrical conductor 120 may be encapsulated or embedded inside the carrier 102. The first mount 101 is located on the underside 102b of the carrier 102. The first mount 101 may comprise an adhesive layer extending all across our at least over a dedicated portion of the underside 102b of the carrier 102. The adhesive layer may comprise or may be constituted by an adhesive agent applied on at least a portion of the underside 102b.

On the upper side 102a there is provided the second mount 110. The second mount 110 is connected to the carrier 102 or is integrally formed with the carrier 102. The second mount 110 may comprise a rigid mounting base 112 which is separately illustrated in Fig. 8. The mounting base 112 comprises a foot 114 and a neck 115. The neck extends on an upper side 114a of the foot 114. The rigid mounting base 112 further comprises a head 116 connected to an end of the neck 115 facing away from the foot 114. As illustrated in Fig. 8 the head 116 comprises a diameter or cross-section at least in one direction or dimension that is larger than a corresponding diameter or cross-section of the adjacently-located neck 115 in that direction or dimension.

The foot 114 comprises a plane-shaped underside 114b that is attached or fixed to the carrier 102. The underside 114b may be adhesively attached to the carrier 102 or may be welded to the carrier 102. For instance, the foot may be connected to the carrier by at least one of ultrasonic welding, mirror welding or warm stacking. The rigid mounting base 112 may comprise an injection molded plastic material whereas the carrier 102 may comprise a pliable plastic foil.

In the configuration of the mounting adapter 100 as illustrated in Fig. 7 the carrier 102 is covered by a cover layer 106. The cover layer 106 comprises a geometry that is substantially identical to the geometry of the carrier 102. The cover layer 106 may also comprise a pliable plastic foil or a paper-based label. The cover layer 106 may be adhesively attached to the upper side 102a of the carrier 102. The cover layer 106 may be of an electrically insulating sheet material. The cover layer 106 covers and electrically insulates the electrical conductor 120 when attached to the carrier 102. Here, at least one of the upper side 102a and an underside 106b of the carrier 102 and the cover layer 106 may be provided with an adhesive thus enabling an adhesive attachment of the cover layer 106 to the carrier 102.

In the region of the second mount 110 the cover layer 106 comprises a recess 106c extending into a side edge of the cover layer 106. The recess 106c comprises a longitudinal slit 160 having a width 161 perpendicular to its elongation that matches with a respective thickness or cross-sectional dimension of the neck 115 of the rigid mounting base 112 perpendicular to the elongation of the longitudinal slit 160 or perpendicular to the elongation of the elongated neck 115. In this way, the slit 160 is configured to slidably receive the neck 115. The neck 115 can be introduced from the side edge of the cover layer 106 and into the recess 106 C along a mounting direction M. The width 161 is narrower or smaller than the outer dimensions of the foot 114. In this way and when adhesively attached or bonded to the carrier 102 the cover layer 106 helps to fasten the rigid mounting base 112 to the carrier 102.

The cover layer 106 may comprise a printed label configured for an adhesive attachment to the housing 10 of the injection device 1. The printed label is typically provided on an upper side 106a of the cover layer 106. Such a label may contain or provide visual instructions, e.g. in form of pictures, symbols or text that relate to the medicament located inside the injection device and its way of use.

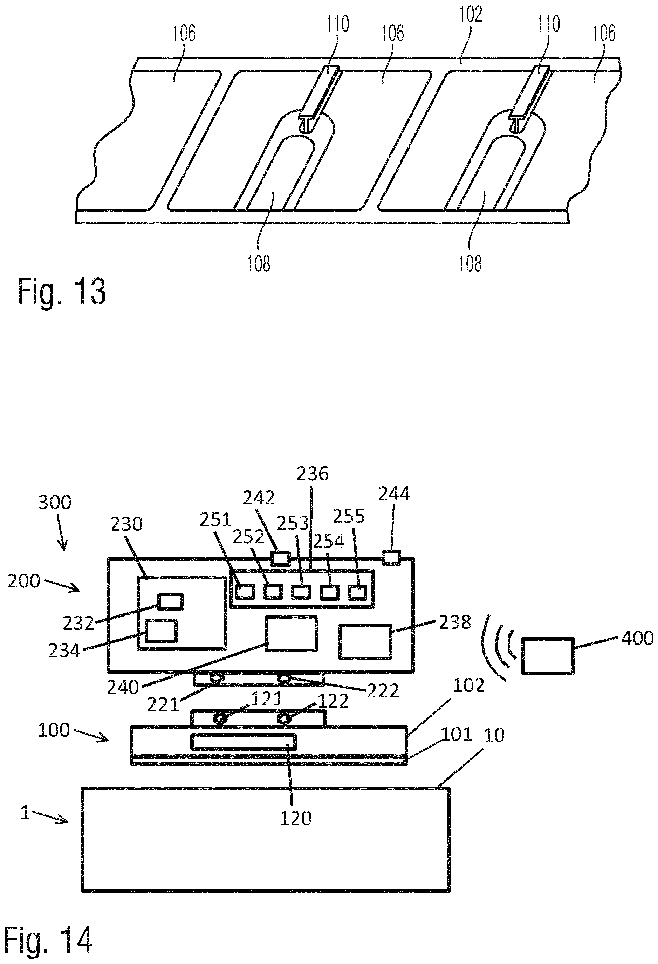

As further illustrated in Fig. 13 the mounting adapter 100 may be manufactured in a mass production process. The various layers of the multilayered structure of the mounting adapter may be provided on coils or rolls and may be subject to a roll-based printing process. It is particularly conceivable, that the carrier 102 and optionally at least one of the electric layer 104 and the cover layer 106 are initially provided on a reel, drum or coil. The production of the mounting adapter may only require minor modifications in an existing mass production process for printing labels for injection devices, such as injection pens. The cover layer 106 may comprise a label section 108 in which medicament related or device related information may be provided or printed. The label section 108 may be enclosed by an arc -shaped, rectangular shaped, circular shaped or oval-shaped enclosure, thus visually emphasizing the information content of the label section 108.

The underside of the carrier 102 may be provided with an adhesive or with an adhesive layer. Here, the adhesive layer may form or constitute the first mount 101. This adhesive layer may be covered with a detachable foil in order to enable a non-stick handling of the mounting adapter 100 before attaching the same to the housing 10 of the injection device 1. Prior to an adhesive attachment of the mounting adapter 100 to the housing 10 it is required that the foil covering the adhesive layer is detached from the housing adapter 100, thus exposing the adhesive layer and/or the first mount 101.

In another example of the mounting adapter 100 the mounting adapter is additionally provided with an electric layer 104. The electric layer 104 comprises a sheet of an electrically insulating foil that is arranged on the upper side 102a of the carrier 102. It is arranged between the cover layer 106 and the carrier 102. The electric layer 104 may comprise a pliable plastic and hence electrically insulating foil that is provided with a printed electrical conductor. In particular, a least one or several conductor loops may be printed on the electric layer.

The electrical conductor 120, e.g. a first electrical conductor 120 may be located or printed on the upper side 104a of the electric layer 104. A second electrical conductor may be arranged and/or printed on an opposite, hence on an underside 104b of the electric layer 104. The electric conductors 120 provided on opposite sides of the electric layer 104 may be electrically insulated or electrically connected depending on the type of implementation of the respective electrical conductors 120. The electric layer 104 may equally comprise a recess 104c as described in connection with Fig. 7. The recess 104c may overlap with the recess 106c.

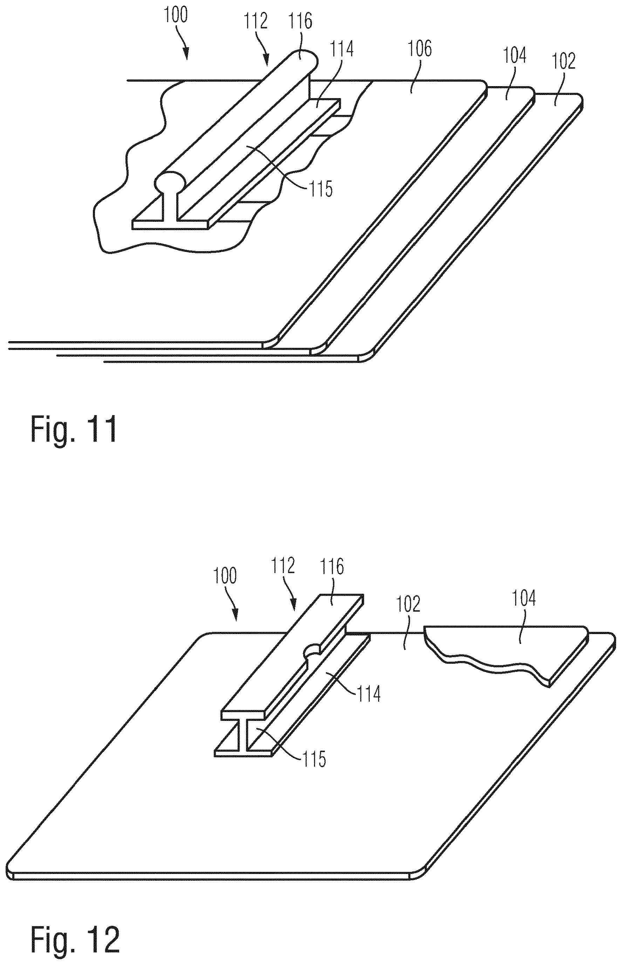

In Fig. 12 another example of a rigid mounting base 112 is illustrated. This mounting base also has a widened elongated foot 114, a narrowing or narrowed neck 115 and a widened head 116. The cross-sectional profile of the rigid mounting base 112 as illustrated in Fig. 12 is of double T-shape.

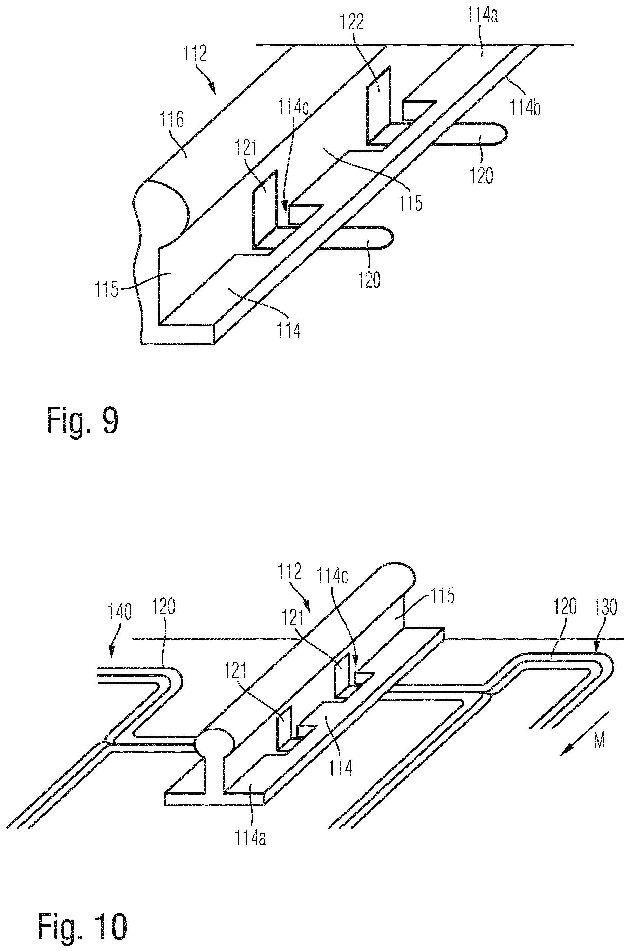

With the examples of Figs. 9 and 10 it is illustrated, that the mounting adapter 100 comprises at least a first electrical contact 121. The mounting adapter 100 comprises at least a first electrical contact 121 and a second electrical contact 122. The electrical contacts 121, 122 are electrically or conductively connected to respective electric conductors 120 as illustrated in Fig. 10.

In examples, wherein the second mount 110 is arranged on top of the carrier 102 and/or on top of an optional electric layer 104 the second mount 110, in particular its rigid mounting base 112 comprises at least one recess or a through opening 114c extending through the structure of the rigid mounting base 112. Here, the electrical conductor 120 extends through the through opening 114c of the foot 114 so that respective electrical contacts 121, 122 can be provided on an upper side 114a of the foot 114 of the rigid mounting base 112. As illustrated in Figs. 9 and 10 the electrical contacts 121, 122 that are located at terminal ends of respective electrical conductors or at opposite ends of a conductor loop are provided at a sidewall section of the neck 115 of the rigid mounting base 112.

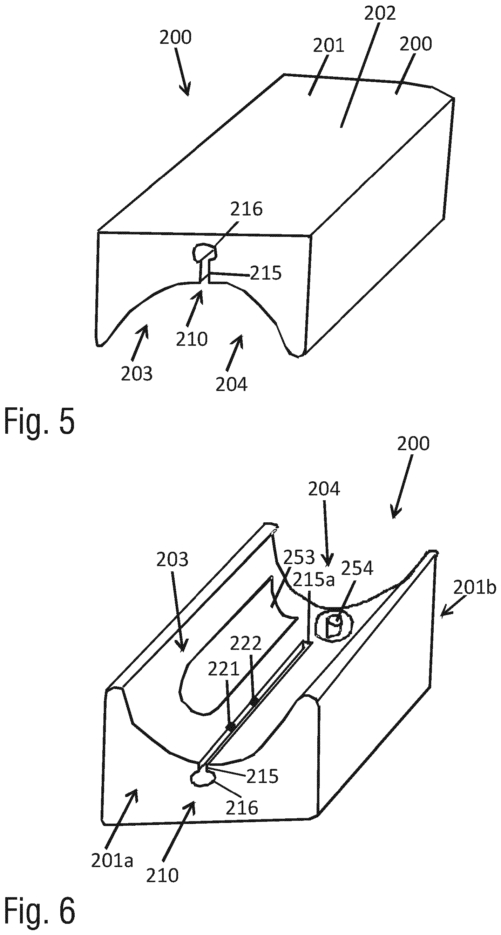

In longitudinal direction, hence along a mounting direction M as illustrated in Fig. 10 the electrical contacts 121, 122 are located at dedicated or predetermined positions. The add-on device 200 as illustrated in Figs. 5 and 6 comprises correspondingly-shaped electrical device contacts 221, 222 that get in mechanical and electrically conducting contact with respective electrical contacts 121, 122 of the mounting adapter 100 when the add-on device 200 is appropriately mounted to the mounting adapter 100.

As illustrated in Fig. 6, the electrical device contacts 221, 222 are located on the third mount. The third mount is complementary-shaped to the second mount as illustrated in Fig. 10. It comprises a head portion 216 and an adjacently-located neck portion 215. The neck portion 215 and the head portion 216 as well as the neck 115 and the head 116 comprise a constant cross-section or profile along the mounting direction M. In this way the third mount 210 can be assembled and attached to the second mount 110 by way of a longitudinal sliding motion of the add-on device 200 relative to mounting adapter 100 along the mounting direction M. The neck portion 215 may form a longitudinal groove in the undersides 203 of the housing 201 of the add-on device 200.

The neck portion 215 may be open ended towards a front face 201a of the housing 201 pointing in distal direction 2 when correctly attached to the injection device 1. At the opposite end and hence in proximal direction 3 the neck portion 215 terminates at a stop 215a. The stop 215a is located at a predefined distance from an opposite end face 201b of the housing. When appropriately assembled to the mounting adapter 100 and when a proximal edge or proximal end face of the mounting base 112 gets in axial abutment with the stop 215a the mutually corresponding electrical contacts 121, 221 and 122, 222 are electrically connected. in other examples the neck portion 215 maybe open towards the proximal end, hence towards the end face 201b while it is closed or blocked by a corresponding stop towards the distal end face 201a.

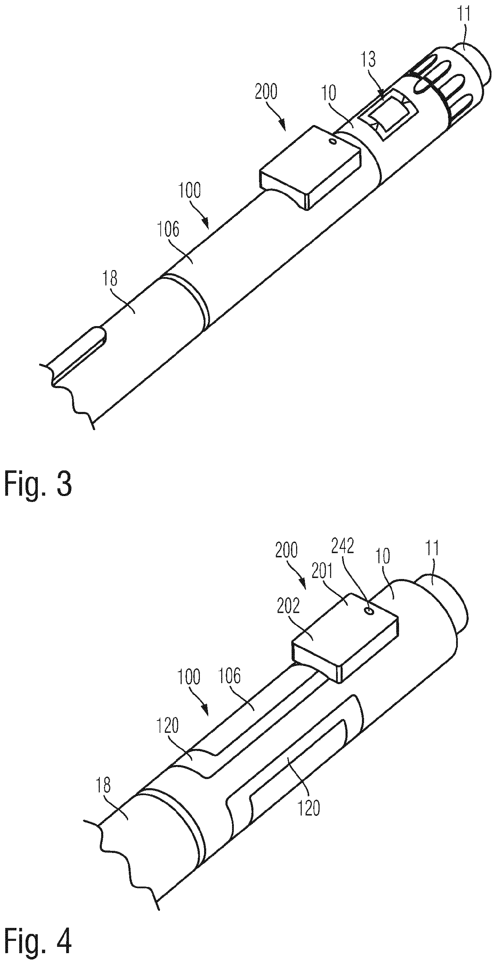

When reaching a final assembly configuration as for instance illustrated in Figs. 3 and 4 the electrical contact 121 is electrically connected to the electrical device contact 221 and the electrical contact 122 is electrically connected to the electrical device contact 222.

The add-on device 200 as illustrated in Figs. 5 and 6 comprises a rigid housing 201 to enclose various electronic components of the add-on device 200. The housing 201 comprises an upper side 202 facing away from the housing 10 of the injection device when assembled to the injection device 1 via the mounting adapter 100. The housing 201 further comprises an underside 203 opposite to the upper side 202. As illustrated, the third mount 210 is located on the underside 203. When the add-on device 200 is connected to the mounting adapter 100 it entirely covers the second mount 110. The underside 203 may comprise an arc -shaped semi-tubular structure 204 configured to receive a portion of an outside surface of the housing 10 of the injection device 1.

On the underside 203 there may be optionally provided a planar-shaped sensor, such as a capacitive sensor 253. Additionally or alternatively there may be provided an optical sensor 254, such as a photodiode or a camera. Additionally or alternatively there may be provided a mechanical switch protruding from the underside 203. Upon mounting the add-on device 200 to the mounting adapter and hence to the injection device 1 the switch may be depressed thus turning on the add-on device or setting the add-on device 200 at least in a standby mode.

In Fig. 14 a block diagram of an injection system 300 is illustrated. The injection system 300 comprises a mounting adapter 100 connectable or connected to an injection device 1 and an add-on device 200 connected to the mounting adapter 100. The mounting adapter 100 is connectable to the housing 10 of the injection device 1 by means of the first mount 101 located on the underside 102b of the carrier 102. From the opposite side, hence from the upper side 102a of the carrier 102 there protrudes the second mount 110. The second mount 110 is correspondingly-shaped to the third mount 210 of the add-on device 200. In the region of the mounts 110, 210 there are provided mutually corresponding electrical contacts 121, 122, 221, 222.

As further indicated in Fig. 14 the add-on device 200 comprises at least a processor 232 and a memory 234. The processor 232 and the memory 234 may be located or arranged on a common printed circuit board 230. There may be further provided a sensor arrangement 236. The sensor arrangement may comprise at least one of an acceleration sensor 251, a position sensor 252, a capacitive sensor 253, an optical sensor 254 and a magnetic sensor 255. The sensor arrangement 236 may further comprise a flexible foil serving as a carrier or support for at least one of the acceleration sensor 251, the position sensor 252, the capacitive sensor 253, the optical sensor 254 and the magnetic sensor 255. The flexible foil may be configured for coiling around the housing 10.

The add-on device 200 may further comprise a user interface 242. The user interface 242 may comprise at least a visual indicator, such as a single color or multicolor light emitting diode (LED). Alternatively, the user interface may comprise a display, such as a liquid crystal display or a two-dimensional display or screen, e.g. a touch sensitive display. In addition, the add-on device 200 may comprise an actuation element 244, e.g. in form of a push button or a rotatable wheel or element. The actuation element 244 may be configured to induce a wireless communication to an external electronic device 400 configured to communicate with the add-on device 200 wirelessly.

The add-on device may comprise a communication unit 238 configured to establish a wireless communication link to the external electronic device 400. Typically, the add-on device 200 further comprises an electric power source 240, e.g. in form of a battery. The electric power source 240 may be only optional. It is conceivable, that the electrical conductor 120 comprises or forms an antenna that is configured to harvest or to capture electric power and hence electric energy from a surrounding electromagnetic field, that may be supplied or provided by the external electronic device 400. In this way, the add-on device 200 may be implemented as a passive electronic device that requires interaction with an external electromagnetic field in order to exchange data. It is conceivable, that the electric power source 240 is a rechargeable battery that can be regularly charged when the add-on device establishes an energy transferring or data transferring wireless link to the external electronic device 400.

In this way, lifetime and/or storage capacity of the electric power source 240 can be effectively prolonged.

The processor 232 and its interaction with at least one or several of the sensors 251, 252, 253, 254, 255 of the sensor arrangement 236 may be configured to enable a gesture recognition, i.e. to identify various handling steps that are conducted by a user in the course of preparing, setting and dispensing a dose of the medicament. For instance with the help of the acceleration sensor and/or a position sensor each handling step of the injection device 1, e.g. removing of one of the caps 18, 17, 16 can be detected. Typical acceleration patterns recorded over time can be identified and can be combined with further information, such as points of time at which the respective accelerations or forces applied to the injection device 1 have been measured.

In this way, safety conclusions about a performed user step can be drawn from the data delivered by the various sensors 251, 252, 253, 254, 255. By means of at least one of an acceleration sensor and a position sensor the gripping of the injection device, the removing of a cap, turning over the injection device, the piercing of the skin prior to an injection procedure, the injection of a dose, the removal of the injection device from the skin and the attachment of a cap to the device can be identified. By means of a suitable position sensor the attachment of a needle assembly 15 to the injection device as well as setting and injecting of a dose can be determined.

The processor 232 and its interaction with at least one or several of the sensors 251, 252, 253, 254, 255 of the sensor arrangement 236 may be also configured to detect a start of an injection procedure, e.g. when a piston rod 9 of the drive mechanism 8 of the injection device 1 gets in abutment with the bung 7 of the cartridge 6.

The position sensor may be based on a capacitive, optical, electrical or magnetic sensor principle. All of the above mentioned handling steps of the injection device can be assigned with a timestamp. If the processor 232 of the add-on device 200 should detect, that a consecutive timestamps between at least two selected identified actions or handling steps are below or above a predefined threshold the processor 232 may be configured to generate at least one of an audible, haptic or visual alert signal via the user interface 242.

Identification of various steps of device handling may be based on a series of predefined signal patterns of the various sensors 251, 252, 253, 254, 255. Moreover, since every user or patient may be characterized by slightly different handling steps the processor 232 and the memory 234 may be switchable into a learn or teaching mode. Here, the user or patient may train an automatic handling step recognition.

The acceleration sensor 251 may comprise or form a non-contact sensor. The acceleration sensor 251 may be disposed inside or the surface of the add-on device 200. Depending on the sensor technology used, a transparent window may cover the acceleration sensor 251 to prevent contamination and damage.

The acceleration sensor 251 may be configured to output signals indicative of the change in velocity of a user's hand relative to the sensor 251 as the user conduct a handling step of the injection device 1. For instance, the output of the sensor 251 may be indicative of detaching or removing 1 of the caps 16, 17 or 18. The acceleration sensor 251 may be referred to as a non-contact sensor, since it is able to sense the change in velocity of a user's hand without contact between the sensor 251 and the user's hand.

In some examples the memory 234 is a separate component, while in other embodiments this memory 234 is integral with the processor 232 or integral with the acceleration sensor 251. The processor 232 may comprise a low power processor 232. It may be preprogramed with at least one acceleration profile or may have access to at least one acceleration profile indicative of a typical handling step of the injection device, such as a typical cap removal movement.

The processor 232 may be configured to determine the change in velocity of the user's hand relative to the acceleration sensor 251. In order to identify a certain handling step of the injection device the processor 232 may the configured to check signals received from the sensor arrangement 236 against one or more acceleration profiles stored in the memory 234. For instance, when the low power processor 232 determines that the received signals are indicative of a typical cap removal movement of the user's hand, it may automatically switch into an activation mode.

Numerous variants of the acceleration profiles described above may also be stored in the memory to account for variations in static friction force or a required pull-off force e.g. between a device component, such as 16, 17, 18 and the injection device 1, and variations in the way a user performs each individual cap removal process.

The optional position sensor 252 may be connected to and at least partially controlled by the processor 232. The position sensor 252 may be on or within the add-on device 200. It may be directed towards a proximal end of the drug delivery device. The position sensor 252 is configured to output signals indicative of the position and/or orientation of the user's hand. For example, it may be possible to determine from the signals output by the position sensor 252, whether the user is using his left hand or right hand to grasp the injection device 1 or a cap 18. The memory 234 may store separate acceleration profiles for left and right hand removal of caps 16, 17, 18 and general device handling. The position sensor 252 may for example be a passive infrared sensor or a capacitive displacement sensor.

The acceleration sensor 251 may take a number of different forms. For example, an electromagnetic sensor which operates based on electromagnetic reflection. The sensor 251 may use optical or infra-red light and rely on the Doppler shift of the light reflected form the moving hand of the user to detect the acceleration. Alternatively, microwaves or radio waves may be used. These have the advantage of being intrinsically lower in energy than visible light, and may pass through an opaque section of the housing 201 of the add-on device 200. Alternatively, the non-contact acceleration sensor 251 may be embodied as a capacitive displacement sensor. The proximity of the user's hand changes the capacitance detected by the sensor. In some other embodiments a capacitive displacement sensor may be used as a combined non-contact acceleration sensor 251 and absolute position sensor 252.

The add-on device 200 may also be placed into a machine learning mode in which the acceleration profiles can be created or updated. For example a user or technician may use the user interface 242 to enter a non-contact acceleration sensor learning mode. The user or technician may then repeatedly remove and replace 1 of the caps 16, 17 or 18 under instruction form the user interface 242 of the add-on device 200. In this way, the add-on device 200 or supplementary device 200 can refine the acceleration profile to better match the real world situation, or create new acceleration profiles which better match the way in which a particular operates the injection device 1.

### List of reference numbers

- 1: injection device
- 2: distal direction
- 3: proximal direction
- 4: first sense of rotation
- 5: second sense of rotation
- 6: medicament container
- 7: bung
- 8: drive mechanism
- 9: piston rod
- 10: housing
- 11: trigger
- 12: dose dial
- 14: cartridge holder
- 15: needle assembly
- 16: inner needle cap
- 17: outer needle cap
- 18: protective cap
- 20: threaded socket
- 100: mounting adapter
- 101: mount
- 102: carrier
- 102a: upper side
- 102b: underside
- 104: electric layer
- 104a: upper side
- 104b: underside
- 104c: recess
- 106: cover layer
- 106a: upper side
- 106b: underside
- 106c: recess
- 108: label section
- 110: mount
- 112: mounting base
- 114: foot
- 114a: upper side
- 114b: underside
- 114c: through opening
- 115: neck
- 116: head
- 120: electrical conductor
- 121: electrical contact
- 122: electrical contact
- 130: capacitive electrode
- 140: antenna
- 160: slit
- 161: width
- 200: add-on device
- 201: housing
- 201a: end face
- 201b: end face
- 202: upper side
- 203: underside#
- 204: semi-tubular shaped structure
- 210: mount
- 215: neck portion
- 215a: stop
- 216: head portion
- 221: electrical device contact
- 222: electrical device contact
- 230: printed circuit board
- 232: processor
- 234: memory
- 236: sensor arrangement
- 238: communication unit
- 240: electrical power source
- 242: user interface
- 244: actuation element
- 251: sensor
- 252: sensor
- 253: sensor
- 254: sensor
- 255: sensor
- 300: injection system
- 400: electronic device

## Claims

1. A mounting adapter configured for mounting an add-on device (200) to an injection device (1), the mounting adapter (100) comprises:
- a carrier (102),
- a first mount (101) connected to the carrier (102) and configured to connect to a housing (10) of the injection device (1),
- a second mount (110) connected to the carrier (102) and configured to connect to the add-on device (200), **characterized by**
- an electrical conductor (120) arranged in or on the carrier (102), wherein the electrical conductor (120) is configured to exchange at least one type of electrical signals with the add-on device (200), and
- at least a first electrical contact (121, 122) connected to the electrical conductor (120), wherein the at least first electrical contact (121, 122) is arranged on at least one of:
- the second mount (110),
- an upper side of the carrier (102),
- an upper side of an electric layer (104) arranged on the upper side of the carrier (102) and
- an upper side of a cover layer (106) extending over a major portion of the upper side of the carrier (102).

2. The mounting adapter according to claim 1, wherein the carrier (102) comprises a sheet of an elastic foil.

3. The mounting adapter according to claim 1 or 2, wherein the first mount (101) comprises an adhesive layer arranged on or extending across an underside of the carrier (102), wherein the underside of the carrier is opposite to the upper side of the carrier (102).

4. The mounting adapter according to any one of the preceding claims, further comprising the electric layer (104) comprising a sheet of an electrically insulating foil arranged on the upper side of the carrier (102), wherein the electrical conductor (120) is arranged on the electrically insulating foil.

5. The mounting adapter according to any one of the preceding claims, further comprising the cover layer (106) extending over the major portion of the upper side of the carrier (102) and covering the electrical conductor (120).

6. The mounting adapter according to any one of the preceding claims, wherein the at least first electrical contact (121, 122) is configured to establish an electrical connection between the electrical conductor (120) and the add-on device (200).

7. The mounting adapter according to any one of the preceding claims, wherein the second mount (110) comprises a rigid mounting base (112) with a foot (114) and a neck (115), wherein the foot (114) is connected to the carrier (102) and wherein the neck (115) extends through at least one of the electric layer (104) and the cover layer (106).

8. The mounting adapter according to any one of the preceding claims, wherein the electrical conductor (120) comprises or constitutes at least one of a measuring electrode (130) and an antenna (140).

9. An add-on device configured for monitoring the operation of an injection device (1), the add-on device (200) comprising a housing (201) with a third mount (210) configured to mechanically engage with the second mount (110) of a mounting adapter (100) according to any one of the preceding claims, **characterized by** at least a first electrical device contact (221, 222) arranged on at least one of the housing (201) and the third mount (210) and being configured to electrically connect with the at least first electrical contact (121, 122) of the mounting adapter (100), when the add-on device (200) is connected to the mounting adapter (100).

10. The add-on device according to claim 9, further comprising a memory (234) and a processor (232) connected to the at least first electrical device contact (221, 222).

11. The add-on device according to any one of the preceding claims, further comprising a sensor arrangement (236), the sensor arrangement (236) comprising at least one of an acceleration sensor (251), a position sensor (252), a capacity sensor (253), an optical sensor (254) and a magnetic sensor (255).

12. The add-on device according to any one of the preceding claims 9 to 11, further comprising a communication unit (238) connected to the at least first electrical device contact (221, 222), wherein the communication unit (238) is configured to transceive at least one of electromagnetic signals and electromagnetic energy via the at least first electrical conductor (120) of the mounting adapter (100) when the at least first electrical contact (121, 122) is connected to the at least first electrical device contact (221, 222).

13. An injection system comprising:
- a mounting adapter (100) according to any one of the preceding claims 1 to 8,
- an add-on device (200) according to any one of the preceding claims 9 to 12, and
- an injection device with a housing,
wherein the first mount (101) of the mounting adapter (100) is connected to the housing (10) of the injection device (1) and wherein the third mount (210) of the add-on device is connected to the second mount (210) of the mounting adapter (100).

14. The injection system according to claim 13, wherein the injection device comprises a medicament container (6) filled with an injectable medicament.

## Patentansprüche

1. Montageadapter, der zum Montieren einer Zusatzvorrichtung (200) an einer Injektionsvorrichtung (1) ausgestaltet ist, wobei der Montageadapter (100) aufweist:
- einen Träger (102),
- eine erste Halterung (101), die mit dem Träger (102) verbunden ist und ausgestaltet ist, um mit einem Gehäuse (10) der Injektionsvorrichtung (1) verbunden zu werden,
- eine zweite Halterung (110), die mit dem Träger (102) verbunden ist und ausgestaltet ist, um mit der Zusatzvorrichtung (200) verbunden zu werden, **gekennzeichnet durch**
- einen elektrischen Leiter (120), der in oder auf dem Träger (102) angeordnet ist, wobei der elektrische Leiter (120) ausgestaltet ist, um mindestens einen Typ von elektrischen Signalen mit der Zusatzvorrichtung (200) auszutauschen, und
- zumindest einen ersten elektrischen Kontakt (121, 122), der mit dem elektrischen Leiter (120) verbunden ist, wobei der zumindest erste elektrische Kontakt (121, 122) auf mindestens einem der folgenden angeordnet ist:
- der zweiten Halterung (110),
- einer oberen Seite des Trägers (102),
- einer oberen Seite einer elektrischen Schicht (104), die auf der oberen Seite des Trägers (102) angeordnet ist, und
- einer oberen Seite einer Abdeckschicht (106), die sich über einen Großteil der oberen Seite des Trägers (102) erstreckt.

2. Montageadapter nach Anspruch 1, wobei der Träger (102) eine Lage einer elastischen Folie aufweist.

3. Montageadapter nach Anspruch 1 oder 2, wobei die erste Halterung (101) eine Klebeschicht aufweist, die auf einer Unterseite des Trägers (102) angeordnet ist oder sich darüber erstreckt, wobei die Unterseite des Trägers der oberen Seite des Trägers (102) gegenüber liegt.

4. Montageadapter nach einem der vorhergehenden Ansprüche, ferner aufweisend die elektrische Schicht (104), die eine Lage einer elektrisch isolierenden Folie aufweist, die auf der oberen Seite des Trägers (102) angeordnet ist, wobei der elektrische Leiter (120) auf der elektrisch isolierenden Folie angeordnet ist.

5. Montageadapter nach einem der vorhergehenden Ansprüche, ferner aufweisend die Abdeckschicht (106), die sich über den Großteil der oberen Seite des Trägers (102) erstreckt und den elektrischen Leiter (120) bedeckt.

6. Montageadapter nach einem der vorhergehenden Ansprüche, wobei der zumindest erste elektrische Kontakt (121, 122) ausgestaltet ist, um eine elektrische Verbindung zwischen dem elektrischen Leiter (120) und der Zusatzvorrichtung (200) zu errichten.

7. Montageadapter nach einem der vorhergehenden Ansprüche, wobei die zweite Halterung (110) eine starre Montagebasis (112) mit einem Fuß (114) und einem Hals (115) aufweist, wobei der Fuß (114) mit dem Träger (102) verbunden ist und wobei sich der Hals (115) durch mindestens eine von der elektrischen Schicht (104) und der Abdeckschicht (106) hindurch erstreckt.

8. Montageadapter nach einem der vorhergehenden Ansprüche, wobei der elektrische Leiter (120) mindestens eine von einer Messelektrode (130) und einer Antenne (140) aufweist oder bildet.

9. Zusatzvorrichtung, die zum Überwachen des Betriebs einer Injektionsvorrichtung (1) ausgestaltet ist, wobei die Zusatzvorrichtung (200) ein Gehäuse (201) mit einer dritten Halterung (210) aufweist, die ausgestaltet ist, um mechanisch in Eingriff mit der zweiten Halterung (110) eines Montageadapters (100) gemäß einem der vorhergehenden Ansprüche zu kommen, **gekennzeichnet durch** zumindest einen ersten elektrischen Vorrichtungskontakt (221, 222), der auf mindestens einem von dem Gehäuse (201) und der dritten Halterung (210) angeordnet ist und ausgestaltet ist, um elektrisch mit dem zumindest ersten elektrischen Kontakt (121, 122) des Montageadapters (100) verbunden zu sein, wenn die Zusatzvorrichtung (200) mit dem Montageadapter (100) verbunden ist.

10. Zusatzvorrichtung nach Anspruch 9, ferner aufweisend einen Speicher (234) und einen Prozessor (232), der mit dem zumindest ersten elektrischen Vorrichtungskontakt (221, 222) verbunden ist.

11. Zusatzvorrichtung nach einem der vorhergehenden Ansprüche, ferner aufweisend eine Sensoranordnung (236), wobei die Sensoranordnung (236) mindestens einen von einem Beschleunigungssensor (251), einem Positionssensor (252), einem Kapazitätssensor (253), einem optischen Sensor (254) und einem magnetischen Sensor (255) aufweist.

12. Zusatzvorrichtung nach einem der vorhergehenden Ansprüche 9 bis 11, ferner aufweisend eine Kommunikationseinheit (238), die mit dem zumindest ersten elektrischen Vorrichtungskontakt (221, 222) verbunden ist, wobei die Kommunikationseinheit (238) ausgestaltet ist, um mindestens eines von elektromagnetischen Signalen und elektromagnetischer Energie über den zumindest ersten elektrischen Leiter (120) des Montageadapters (100) zu senden bzw. zu empfangen, wenn der zumindest erste elektrische Kontakt (121, 122) mit dem zumindest ersten elektrischen Vorrichtungskontakt (221, 222) verbunden ist.

13. Injektionssystem, aufweisend:
- einen Montageadapter (100) nach einem der vorhergehenden Ansprüche 1 bis 8,
- eine Zusatzvorrichtung (200) nach einem der vorhergehenden Ansprüche 9 bis 12 und
- eine Injektionsvorrichtung mit einem Gehäuse,
wobei die erste Halterung (101) des Montageadapters (100) mit dem Gehäuse (10) der Injektionsvorrichtung (1) verbunden ist und wobei die dritte Halterung (210) der Zusatzvorrichtung mit der zweiten Halterung (210) des Montageadapters (100) verbunden ist.

14. Injektionssystem nach Anspruch 13, wobei die Injektionsvorrichtung einen Medikamentenbehälter (6) aufweist, der mit einem injizierbaren Medikament gefüllt ist.

## Revendications

1. Adaptateur de montage configuré pour monter un dispositif complémentaire (200) sur un dispositif d'injection (1), l'adaptateur de montage (100) comprenant :
- un support (102),
- un premier montant (101) connecté au support (102) et configuré pour se connecter à un boîtier (10) du dispositif d'injection (1),
- un deuxième montant (110) connecté au support (102) et configuré pour se connecter au dispositif complémentaire (200), **caractérisé par**
- un conducteur électrique (120) agencé dans ou sur le support (102), le conducteur électrique (120) étant configuré pour échanger au moins un type de signaux électriques avec le dispositif complémentaire (200), et
- au moins un premier contact électrique (121, 122) connecté au conducteur électrique (120), l'au moins premier contact électrique (121, 122) étant agencé sur au moins l'un des éléments suivants :
- le deuxième montant (110),
- une face supérieure du support (102),
- une face supérieure d'une couche électrique (104) agencée sur la face supérieure du support (102) et
- une face supérieure d'une couche de recouvrement (106) s'étendant sur une majeure partie de la face supérieure du support (102).

2. Adaptateur de montage selon la revendication 1, le support (102) comprenant une feuille d'une tôle élastique.

3. Adaptateur de montage selon la revendication 1 ou 2, le premier montant (101) comprenant une couche d'adhésif agencée sur ou s'étendant sur une face inférieure du support (102), la face inférieure du support étant opposée à la face supérieure du support (102).

4. Adaptateur de montage selon l'une quelconque des revendications précédentes, comprenant en outre la couche électrique (104) comprenant une feuille d'une tôle isolante électriquement agencée sur la face supérieure du support (102), le conducteur électrique (120) étant agencé sur la tôle isolante électriquement.

5. Adaptateur de montage selon l'une quelconque des revendications précédentes, comprenant en outre la couche de recouvrement (106) s'étendant sur la majeure partie de la face supérieure du support (102) et recouvrant le conducteur électrique (120).

6. Adaptateur de montage selon l'une quelconque des revendications précédentes, l'au moins premier contact électrique (121, 122) étant configuré pour établir une connexion électrique entre le conducteur électrique (120) et le dispositif complémentaire (200).

7. Adaptateur de montage selon l'une quelconque des revendications précédentes, le deuxième montant (110) comprenant une base de montage rigide (112) comportant un pied (114) et un col (115), le pied (114) étant connecté au support (102) et le col (115) s'étendant à travers au moins l'une de la couche électrique (104) et de la couche de couverture (106).

8. Adaptateur de montage selon l'une quelconque des revendications précédentes, le conducteur électrique (120) comprenant ou constituant au moins l'une d'une électrode de mesure (130) et d'une antenne (140).

9. Dispositif complémentaire configuré pour surveiller le fonctionnement d'un dispositif d'injection (1), le dispositif complémentaire (200) comprenant un boîtier (201) comportant un troisième montant (210) configuré pour venir en prise mécaniquement avec le deuxième montant (110) d'un adaptateur de montage (100) selon l'une quelconque des revendications précédentes, **caractérisé par** au moins un premier contact de dispositif électrique (221, 222) agencé sur au moins l'un du boîtier (201) et du troisième montant (210) et qui est configuré pour se connecter électriquement avec l'au moins premier contact électrique (121, 122) de l'adaptateur de montage (100), lorsque le dispositif complémentaire (200) est connecté à l'adaptateur de montage (100).

10. Dispositif complémentaire selon la revendication 9, comprenant en outre une mémoire (234) et un processeur (232) connecté à l'au moins premier contact de dispositif électrique (221, 222).

11. Dispositif complémentaire selon l'une quelconque des revendications précédentes, comprenant en outre un agencement de capteur (236), l'agencement de capteur (236) comprenant au moins l'un parmi un capteur d'accélération (251), un capteur de position (252), un capteur de capacité (253), un capteur optique (254) et un capteur magnétique (255).

12. Dispositif complémentaire selon l'une quelconque des revendications précédentes 9 à 11, comprenant en outre une unité de communication (238) connectée à l'au moins premier contact de dispositif électrique (221, 222), l'unité de communication (238) étant configurée pour transmettre au moins l'un parmi des signaux électromagnétiques et de l'énergie électromagnétique par l'intermédiaire de l'au moins premier conducteur électrique (120) de l'adaptateur de montage (100) lorsque l'au moins premier contact électrique (121, 122) est connecté à l'au moins premier contact de dispositif électrique (221, 222).

13. Système d'injection comprenant :
- un adaptateur de montage (100) selon l'une quelconque des revendications précédentes 1 à 8,
- un dispositif complémentaire (200) selon l'une quelconque des revendications précédentes 9 à 12, et
- un dispositif d'injection comportant un boîtier,
le premier montant (101) de l'adaptateur de montage (100) étant connecté au boîtier (10) du dispositif d'injection (1) et le troisième montant (210) du dispositif complémentaire étant connecté au deuxième montant (210) de l'adaptateur de montage (100).

14. Système d'injection selon la revendication 13, le dispositif d'injection comprenant un récipient de médicament (6) rempli d'un médicament injectable.
